Europäisches Patentamt

European Patent Office

Office européen des brevets

(11) Publication number: **0 392 218**
**A2**

(12) **EUROPEAN PATENT APPLICATION**

(21) Application number: **90105097.1**

(51) Int. Cl.5: **A61L 15/32**

(22) Date of filing: **19.03.90**

(30) Priority: **17.04.89 IT 2015889**

(43) Date of publication of application:
**17.10.90 Bulletin 90/42**

(84) Designated Contracting States:
**AT BE CH DE DK ES FR GB GR IT LI LU NL SE**

(71) Applicant: **Petrescu, Dorin Olimpiu**
**Viale Cassiodoro 4**
**I-20145 Milano(IT)**

Applicant: **Bergamini, Nino**
**Via F.lli Cervi, 10**
**I-20090 Segrate (Résidenza Cedri)(IT)**

Applicant: **Mauro, Marino**
**Piazza Virgilio**
**I-20124 Milano(IT)**

(72) Inventor: **Petrescu, Dorin Olimpiu**
**Viale Cassiodoro 4**
**I-20145 Milano(IT)**
Inventor: **Bergamini, Nino**
**Via F.lli Cervi, 10**
**I-20090 Segrate (Résidenza Cedri)(IT)**
Inventor: **Mauro, Marino**
**Piazza Virgilio**
**I-20124 Milano(IT)**

(74) Representative: **Giambrocono, Alfonso, Dr.**
**Ing. et al**
**Ing. A. Giambrocono & C. S.r.l. Via Rosolino**
**Pilo 19/B**
**I-20129 Milano(IT)**

(54) **Process for the in vitro preparation of artificial haemostatic membranes.**

(57) A process for the in vitro preparation of sterile artificial haemostatic mambranes endowed with improved mouldability and uniformity of appearance and thickness, made from blood plasma not necessarily of transfusion quality, involving treatment of the clot with hydrodispersible substances, and subsequently with a mixture of emollient and dehydrating agent so as to form a product that is then stretched to form a membrane with the above said superior characteristics.

# PROCESS FOR THE IN VITRO PREPARATION OF ARTIFICIAL HAEMOSTATIC MEMBRANES

The present invention concerns a process for the in vitro preparation of artificial haemostatic membranes (AHM) endowed with improved mouldability and uniformity of appearance and thickness, such process being both economical and easily carried out.

Like catgut, the haemostatic membrane is an organic medical-surgical device for use in medicine and surgery, apt to be absorbed by the body, it may be provided in sterile packages and is intended for therapeutic application in various pathological-surgical conditions. It is a thin mouldable fibrin membrane applicable, on its own or with medicaments added, to the bleeding or exuding surfaces of injured tissues (burns or other wounds of various origins) either in normal subjects or with metabolic or haematologic disorders, to stop bleeding.

As it is known, blood coagulation is a spontaneous physiological process which usually requires from 6 to 18 minutes; during that time a fibrin network develops forming the primary barrier to blood loss from blood vessels. There are, however, certain pathological conditions in which the coagulation time is lengthened abnormally, or where coagulation does not quite occur at all. In such cases therapeutic intervention, which may take a variety of forms, is necessary. One treatment consists in the formation of an artificial fibrin network and this may be achieved in essentially two ways, i.e. by local injection of the components necessary to cause the formation of a haemostatic network, or by application of a pre-formed barrier made of fibrin to the injured tissues.

In vivo and in vitro methods yielding artificial fibrin-like products have previously been published and patented. All these methods necessarily depend on the same fundamental process, i.e. the transformation of a colloidal suspension of fibrinogen, either still in plasma or isolated and purified in vitro, into a fibrin clot. In all cases this requires the addition of a coagulating agent such as, forcedly, calcium chloride for citrate-treated plasma or thrombin for purified fibrinogen solutions. The characteristics distinguishing the various methods relate to the quality of the final product, to the simplicity of the preparation process and to its expediency.

The methods employed to form the fibrin clot in situ have as main disadvantage the technical complexity which, accordingly, renders them rather expensive. In fact they require separate purification and preparation of fibrinogen, of thrombin solution and of aprotinin or other anti-fibrinolytic agents. They also require the use of special syringes and, furthermore, electrical apparatus is necessary to heat the reagents immediately prior to their therapeutic application.

There are also complications with regard to the plasma samples from which the fibrinogen is obtained: these must be previously carefully selected in order to reduce the risk of viral contamination. However this risk cannot be completely avoided and it results in the discarding of considerable amounts of plasma.

The in vitro methods yielding plaques, sheets, foams or films, etc. of fibrin, involve the use of flat bottomed containers, generally covered with paraffin or petrolatum, in which the fibrinogen solution of whole plasma is coagulated. The so formed clot is dried, then sterilized by addition of formaldehyde or by boiling. The main disadvantages of these procedures, in addition to those already mentioned regarding the purification of fibrinogen, concern firstly the use of reagents and/or materials that are potentially harmful (paraffin, petrolatum, formaldehyde) which may still be present in the final product and are hardly removed, and secondly in the rather modest quality of the final product which is brittle, inelastic and not mouldable or only slightly so.

Romanian Patent No. 259/41440 granted in 1959 to one of the present inventors describes a method which eliminates some of the above-cited disadvantages. It relates to an in vitro preparative technique which uses whole plasma as starting material and produces fibrin films of satisfactory quality by washing the just formed clot followed by partial dehydration with alcohol containing glycerol. However certain steps in this procedure detract from its utility. Firstly, a high percentage of initially formed clots breaks up, during transfer from the coagulation container to the washing vessel, and is unutilizable. Furthermore the films obtained are not completely satisfactory from the point of view of quality since they are not sufficiently elastic and mouldable, their form is irregular and their thickness variable. Besides, during the final drying step, the films often tear up, so that the resulting product shows perforations of different size.

It has been now surprisingly found an in vitro method which provides the formation of artificial haemostatic fibrin membranes using plasma as starting material, with no need of first isolating the fibrinogen, and thus eliminating all the above-cited disadvantages.

The plasma employed either human or animal does not necessarily have to be of transfusion quality, and this is the first of the many advantages of the present invention as compared with the prior art methods.

The plasma is collected on a sodium citrate or other appropriate anticoagulant, and may be therein kept for 30 days or a little longer at a temperature between 0°C and 4°C, or for some months at temperatures lower than -20°C (freezing). The coagulation process takes place in containers of the chosen shape and size by means of the addition of a calcium chloride solution, whose concentration may vary according to whether the calcium chloride is in crystalline or amorphous form, or of other coagulation agent.

It has been surprisingly found that the breakage of the initial clots can be avoided by treatment with powdered sodium chloride and/or glucose or other water-soluble or anyway water-dispersable absorbing agent in an amount sufficient to completely cover the surface of the clot: by osmosis these substances slowly and uniformly absorb the serum which would otherwise soak the clot rendering it brittle and easily breakable. Such osmotic step also renders the clot easily detachable from the container walls and therefore eliminates the problem of the high percentage of clots unutilizable as a result of tearing during transfer from one container to another.

After eliminating the residual serum not absorbed by the water-soluble absorbing agent by means of washing for 14-22 hours under a water stream and finally distilled water, the clot is squeezed and/or centrifuged and then treated with 96% ethanol (or other dehydrating agent) containing 10-14% v/v of glycerol or other softening agent. The amount of such solution must be sufficient to completely cover the clot. The rinsing process with said substances is repeated one or more times, for at least one hour each. The above said ratio of the amounts of these two alcohols results in a remarkable improvement in the elastic and moulding properties of the clot after its dehydration.

At this point the clot is stretched between two smooth non-sticky or poorly adherent flat or cylindrical surfaces and then allowed to dry. The surfaces may be of silicone or siliconised; of teflon or teflonised; of enamel, stainless steel, glass or porcelain or other material with low adhesion properties, possibly treated prior to the pressing process with a layer of glycerol. The fact of initiating the stretching step after treatment with glycerol leads to a product having a regularity of form and thickness not previously obtainable. The use of non-sticky surfaces, possibly first treated with glycerol or other suitable softening agent, avoids the risk of tears or perforations in the membrane - a very common occurrence in the procedures employed to date.

The next step is drying in a current of air at a maximum temperature of 100°C for 10-30 minutes.

The end product is a semitransparent membrane with almost perfectly smooth surfaces and highly uniform thickness; it is compact, elastic, flexible, resistant to medium mechanical stress, easily manageable and may be sterilised in autoclave at 120°C for 20-30 minutes, or by treatment with antiseptic or antibiotic solutions.

It may be seen from this description that at no point does the process utilise substances different from the ones normally present in the body or harmful to it.

The above-described process provides, therefore, a number of remarkable advantages. It is a simple, easily carried out procedure and does not require elaborate or costly equipment; it may be carried out with equipment normally present in health clinics or blood testing laboratories. The economical advantage of this is apparent therefrom. Furthermore the present process allows the use, as starting material, of past expiry date plasma samples or those suspected of viral contamination, thus reserving high quality plasma for transfusions. Finally the procedure yields sterile artificial haemostatic membranes of high quality which are slowly absorbed by body tissues.

There follows a detailed description of an example of preparation of the haemostatic membranes of the present application. In no way is this example to be intended as limiting the scope of the present invention.

EXAMPLE

Into a teflon-coated metal tray placed on a perfectly horizontal surface a mixture is poured containig nine volumes of plasma derived from human blood, collected, as normally, on an anticoagulant, and one volume of a 2.6% anhydrous calcium chloride solution in distilled water, so as to form a layer of liquid in the tray 11 mm deep.

The mixture is left to stand at 18-22°C for 2-3 hours. A yellowish gelatinous clot, soaked with serum and lightly adhering to the container walls, is thus formed. The clot is now covered with a 1-2 cm thick layer of powdered sodium chloride which absorbs nearly all of the liquid (serum) soaking the clot, and thus facilitates its detachment from the container. The clot is now subjected to an initial rinse with running (tap) water, and then placed in a deeper container where it is maintained under a continuous flow of tap water for 18 hours. The final phase of this washing is performed with distilled water. The clot has now a whitish colour and looks like a fragment of fibrous fascia.

The clot is removed from water and washed twice successively, each for one and a half hours under gentle stirring, in a bath of ethanol containing 10% v/v of glycerol. It is then removed and placed on a teflon-impregnated cloth to drain. The clot is stretched over this cloth by placing a similar cloth over it and rolling it with a cylindrical roller. The membrane thus formed is then laid out on a curved surface, previously covered with a teflon-impregnated cloth coated with a thin layer of glycerol, and fixed in place with threads. It is then dried in this position in a current of air at up to 100°C, a process which takes up to 20 minutes.

At the end of this step the fibrin membrane, once white-opaque and relatively thick (above 0,5 mm), is now almost transparent and is about 0,14-0,16 mm thick. It is cut into 5 x 7 cm slices which are stacked between teflon-impregnated temperature resistant (up to 250°C) leaves and packaged for autoclaving at 120°C for 20 minutes. The containers are then sealed.

## Claims

1. A process for the in vitro preparation of artificial haemostatic membranes, starting from plasma stored on anticoagulating medium and then transformed into a clot by known methods, said clot being processed to obtain a membrane mouldable and packable in sealed containers after sterilization in autoclave at 120°C, such process being characterized in that the human or animal plasma, not necessarily of transfusion quality, is clotted in a container with poorly or non-sticky surfaces; the obtained clot is treated with water-soluble or water-dispersable absorbing agent which soaks by osmosis the remaining serum and allows the detachment of said clot from said container, said clot being then repeatedly washed with water and distilled water for eliminating the traces of remaining serum, and treated with a dehydrating agent or with a softening agent; stretching the isolated clot between two smooth, flat, poorly or non-sticky surfaces.

2. A process according to claim 1, wherein the water-soluble absorbing agent is sodium chloride and/or glucose.

3. A process according to claims 1 and 2, wherein the dehydrating agent is 96% ethanol.

4. A process according to claims 1 to 3 wherein the softening agent is glycerol in an amount of 10-14% v/v.

5. A process according to claims 1 to 4 wherein the clot treated with ethanol and glycerol is stretched with a cylindrical roller between two smooth, poorly or non-sticky surfaces.

6. A process according to claims 1 to 5 wherein the surfaces, bet ween which the clot is stretched consist of glass, porcelain, teflon, teflonated cloth, silicone, poorly or non-sticky, biodegradable plastic materials, enamel, stainless steel.

7. Process according to claims 1 to 6 wherein the surfaces between which the clot is stretched, are treated with glycerol as softening agent.

8. A sterile (artificial) haemostatic mambrane essentially consisting of fibrin, free of substances not naturally present in the human or animal body, and endowed with high elasticity and mouldability and of highly uniform appearance and thickness.